(19) **Europäisches Patentamt**
**European Patent Office**
**Office européen des brevets**

(11) **EP 4 674 279 A1**

(12) **EUROPEAN PATENT APPLICATION**

(43) Date of publication:
**07.01.2026 Bulletin 2026/02**

(21) Application number: **25183743.1**

(22) Date of filing: **18.06.2025**

(51) International Patent Classification (IPC):
*A23K 20/163* (2016.01)    *A23K 50/20* (2016.01)
*A23K 10/30* (2016.01)    *A61K 31/716* (2006.01)
*A61K 36/39* (2006.01)    *A61K 36/48* (2006.01)
*A61P 1/04* (2006.01)

(52) Cooperative Patent Classification (CPC):
(C-Sets available)
**A23K 20/163; A23K 10/30; A23K 50/20;
A61K 31/716; A61K 36/39; A61K 36/48; A61P 1/04**
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **18.06.2024 IT 202400013960**

(71) Applicant: **Martini, Anna**
**10098 Rivoli (TO) (IT)**

(72) Inventor: **Martini, Anna**
**10098 Rivoli (TO) (IT)**

(74) Representative: **Robba, Pierpaolo**
**Interpatent S.R.L.**
**Via Caboto, 35**
**10129 Torino (IT)**

(54) **FOOD COMPOSITION FOR USE IN A METHOD OF PREVENTING THE ONSET OR TREATING THE SYMPTOMS OF GASTRITIS IN EQUINES**

(57)    Composition for use in a method for preventing the onset or treating the symptoms of gastritis in equines, said method comprising a step of administering a therapeutically effective amount of the composition to an equine, wherein the composition comprises: an amount of dehydrated sweet potato; an amount of β-glucan (BG) powder; an amount of dried St. John's wort (Hypericum perforatum) flowers; an amount of fenugreek seed powder (Trigonella foenum-grecum); an amount of carob pod powder (Ceratonia siliqua).

$$IPC = A23K$$

EP 4 674 279 A1

(52) Cooperative Patent Classification (CPC): (Cont.)

C-Sets
**A61K 31/716, A61K 2300/00**

## Description

*Technical Field*

The present invention relates to a food composition for use in a method of preventing the onset or treating the symptoms of gastritis in equines. More particularly, the present invention relates to a food supplement composition providing for a step of administering a therapeutically effective amount of the composition to an equine.

*Prior Art*

Generally, by food supplement composition for animals it is meant a feed which, due to its composition, is sufficient for a daily ration of an animal only if combined with other feeds.

Just like for humans, also for animals, well-being and health are linked to a correct diet and a calm, stress-free life.

Equines, especially horses, are mono-gastric herbivorous animals that have undergone transformations in their habits over the years.

The wild horse lives in a herd and spends most of its time, about nineteen hours, grazing and browsing on the grass. As a result, its stomach is constantly engaged in digestion, so that the production of digestive enzymes and gastric acid is almost uninterrupted.

Horses for tourist or sporting use are instead mainly raised in stables and lead, also from a nutritional point of view, a life very different from the one they would live in nature. Horses housed in stables are in fact forced to spend a large part of their time under fixed stabling conditions, without the possibility of moving freely outdoors or in an enclosure and above all, as far as the object of the present invention is concerned, without being able to browse on the grass continuously.

Horses raised in these conditions very often, for stable needs, receive hay two or three times a day, in addition to portions, sometimes excessive, of concentrated feed. This feeding method causes an increase in gastric acidity during the hours in which the animals do not feed and in which digestion is therefore not necessary. The increase in gastric acidity results in an increased risk of developing pathologies that, if not treated promptly, can evolve into gastric ulcers.

These living conditions can cause the onset of physical and behavioral disorders, as well as psychophysical stress in the animal.

The horse's digestive system is complex and delicate, and the stomach is structured for continuous work. The balance of the digestive system is therefore easily affected by any change in diet, lack of physical exercise, state of health, even emotional, and any administration of drugs.

The horse's stomach is divided into two regions separated by a band called the Margo Plicatus. The upper region, called the fundus, is represented by the non-glandular portion of the stomach, about a third of it, where the plicated margin is less protected; the lower region, or body, is called the glandular portion and covers the remaining part of the stomach, where the mucosa is rich in glands secreting gastric juices but also enzymes capable of protecting the mucosa itself.

It is not surprising, therefore, that most of the lesions, about 80% of the cases, are found in the upper region close to the plicated margin. Physical exercise, in addition to numerous other factors, significantly increases the incidence of the disease because, during normal movement, the gastric contents tend to remain in the lower part of the stomach; when the work becomes intense, however, the abdominal mass presses on the diaphragm, and therefore on the stomach, causing the contents to rise also in the non-glandular area of the stomach itself.

It is therefore very important to take care of the temporal, qualitative and quantitative administration of meals, avoiding leaving the stomach empty for long periods of time, with consequent excess production of acids and aggressive agents that alter the functioning of the stomach itself.

Horses fed diets rich in concentrated feed and little hay, and therefore lacking in fiber, can develop gastrointestinal disorders due to food overload, reduced consumption times and the consequent alteration of the gastrointestinal balance.

These alterations result in inflammation of the horse's gastrointestinal tract, ulcers, colic and the so-called "horse gastritis".

The syndrome commonly called "horse gastritis" has a multifactorial etiology. The most frequent causes of gastritis in horses are certainly the type of diet, stabling management, lifestyle, stress, factors inherent to the breed, as well as the use and/or abuse of anti-inflammatory drugs.

Horses living in physiological conditions close to those found in their natural habitat enjoy a balance between factors protective of the gastric mucosa and factors for the gastric mucosa, which balance ensures the maintenance of gastric homeostasis.

However, when external factors, such as those mentioned above, come into play, the situation can change and trigger various digestive tract diseases, including digestive tract ulcers. One of the main causes of digestive tract ulcers in horses is excess stomach acid, as is the case in humans and other animals. Excess stomach acid can deteriorate the protective mucosa of the stomach, thus damaging the inner surface of the stomach and causing gastric ulcers.

This pathology can indifferently affect young foals or adult horses, with clinical signs ranging from mild to more serious, such as in the case of deep ulcerations.

The main symptoms of gastric ulcers in horses are: loss of appetite, poor chewing, nervousness and sudden changes in mood during meals and/or normal activity, scratching in the box, rough and dull hair, poor ability to work, loss of body weight, poor performance. In foals, episodes of dysentery, bruxism (continuous grinding of the teeth) and intense salivation add to the above. Recurrent colic may also appear.

For the treatment of gastric ulcers in horses, there are drugs on the market that have omeprazole as their active ingredient, which is able to block the proton pump and therefore reduce the production of gastric acidity, starting the healing process of gastritis. In the long term, the use of drugs based on omeprazole can cause rather severe side effects such as, for example, poor absorption and consequent deficiency of vitamin B12, magnesium and calcium, all important for the musculoskeletal system. Furthermore, by modifying the pH of the stomach, intestinal infections caused by various bacteria are favored.

Drugs specific for gastritis are also known, based on alkalinizing and/or buffering agents, but their prolonged use negatively interferes with the entire metabolism of the horse itself, in addition to their being very expensive.

The use of drugs based on histamine type-2 (H2) receptor antagonists such as cimetidine and ranitidine, both of which reduce the production of gastric acid in the stomach in horses, is also known. Treatment with H2-antagonists is quite effective for the resolution of gastric lesions. However, this treatment requires a dosage that varies considerably from subject to subject, resulting in difficulty in ensuring correct administration.

The above-mentioned pharmacological treatments have a curative effect on gastric ulcers and lesions of the digestive system, but they are not suitable for obtaining a preventive effect on these pathologies.

For these gastric pathologies, there are also natural treatments on the market that are able to restore the correct functioning of the gastric mucosa.

A natural remedy for the treatment of gastric ulcers is given by food charcoal. Food charcoal is odorless and tasteless, therefore easy to be administered to horses, it is pressed without synthetic additives and is also excellent in case of sensitive and delicate stomachs. In fact, natural charcoal contributes to the elimination of elements that weigh down the digestive system and can be an excellent support after a pharmacological treatment.

There are also supplement feeds containing probiotics, buffering substances and fermentation products, which can help reduce inflammation and support the restoration of gastric mucosal integrity. These supplement feeds, when used in conjunction with appropriate pharmacological treatment, can help prevent gastrointestinal disorders in horses.

Other supplement feeds, instead, contain extracts of medicinal plants capable of strengthening the protective film of the gastric mucosa, alleviating heartburn and stimulating and modulating the animal's immune system.

These supplement feeds, although they do not present any contraindications to their use, are generally not palatable to animals, making their administration difficult.

Document US2006008509A1 discloses a dietary supplement specifically formulated to treat and/or prevent digestive tract ulcers in horses and other animals.

The dietary supplement described is composed of a mixture of polar lipids, preferably oat oil, soluble fiber and nutricines.

Polar lipids have the function of strengthening the intestinal mucosa lining the inner wall of the stomach, the soluble fiber acts to slow down the flow of the food ingested with the dietary supplement so as to keep the stomach full for a longer period, while nutricines revitalize the integrity of the membranes of the digestive tract.

Horses, unlike humans, do not have a gallbladder, which is an organ attached to the liver and responsible for storing bile. The gallbladder is essential for the digestion of fats, helping to break down fats into smaller particles that can be absorbed by the intestines. The use of this dietary supplement consisting of a high lipid concentration cannot be prolonged over time as the excess of lipids, particularly polar lipids, can negatively affect the metabolism of horses as it can interfere with the digestion and absorption of nutrients, compromising various cellular functions including insulin resistance and consequently reducing the horse's immune capacity to respond to infections.

Document DE202014002107U1 discloses a supplement feed for the feeding of horses that is used to counteract the development and persistence of gastric mucosal pathologies and is composed of a mixture of vegetal components and one or more magnesium compounds.

The above-mentioned complementary feed consists of the following plant components: chamomile, licorice root, mallow, marshmallow and fenugreek.

Prolonged use or a high quantity of the aforementioned supplement feed containing magnesium could cause diarrhea and dysentery in horses, and particularly in elderly subjects, or in subjects with kidney problems, it could cause a worsening of the pathology as the kidneys have a fundamental role in the excretion of this mineral. In addition, prolonged and heavy use of licorice can cause adverse effects in horses, including increased blood pressure, cardiac failure caused by electrolyte imbalances, metabolic problems due to sugar metabolism, and diarrhea and colic in subjects with pre-existing gastrointestinal sensitivities.

In the light of the above, there is a need to have an animal feed made up of natural elements that is able both to treat the acute phases of inflammation of the gastric walls of animals, especially equines and even more particularly horses, and to prevent onset thereof, as well as to provide a complementary feed the prolonged use of which does not cause unwanted side effects on the health of the horse.

The Applicant has been able to experimentally appreciate that the technical problem highlighted above can be solved through the use of a composition comprising a mixture of natural ingredients in powder form, easy to administer and with both a curative and preventive effect on gastritis and gastric ulcers in horses in general.

The object of the present invention is therefore to overcome the drawbacks of prior art by providing a food supplement composition for animals suitable for use as feed to complete the nutritional profile of an animal's daily requirement.

The object of the present invention is to solve the aforementioned problem, by creating a supplement feed made up of natural ingredients, appropriately balanced with one another in order to obtain an optimal food supplement composition for the effective treatment of gastric pathologies and inflammation of the gastrointestinal tract of horses.

Another object of the present invention is to create a highly effective food supplement composition that allows for optimal weight gain in horses.

A further object of the present invention is to provide a food supplement composition for animals that is digestible and palatable for the animal.

These and other objects are achieved with the food supplement composition according to the invention as claimed in the appended claims.

Further advantages and features of the present invention will become apparent from the following detailed description of some preferred embodiments of the invention, given by way of non-limiting examples.

*Disclosure of the Invention*

The food supplement composition for equines according to the invention mainly comprises:

- an amount of powder of dehydrated sweet potato;
- an amount of powder of β-glucan (BG);
- an amount of powder of dried St. John's wort (Hypericum perforatum) flowers;
- an amount of powder of fenugreek (Trigonella foenum-grecum) seeds;
- an amount of powder of carob (Ceratonia siliqua) pods.

The above ingredients are advantageously used in powder form, thus forming a homogeneous mixture, in order to facilitate absorption.

In a preferred embodiment, the mixture has a typically beige/brown color.

Optionally, the mixture may comprise an amount of flaxseed powder.

Sweet potato (Ipomoea batatas), which is one of the ingredients of the food supplement composition according to the invention, is a low-calorie food (about 85 kcal per 100 grams of product), with a good fiber content (which aids digestion and intestinal function) and a high starch content, which once digestion has begun, tends to form a protective film on the gastric and intestinal mucosa. Furthermore, sweet potato is rich in beta-carotene, which is a precursor of vitamin A, and has important anti-inflammatory properties which, in addition to the presence of vitamins C and B, have a calming effect on gastric ulcers.

Advantageously, thanks to the fact that sweet potato is used in the form of dehydrated powder, the quantity of sugars present is reduced, allowing increasing the dose that can be administered.

The food supplement composition according to the invention further comprises an amount of powder of β-glucan (BG). β-glucans (BGs) are polysaccharides, i.e. complex sugars present in the cell walls of fungi, yeasts, algae, lichens and cereals, such as oats, bran and barley, from which they can be extracted. The differences between the different types of BGs depend on their chemical structure; they are all complex sugars made up of glucose units, which however can be linked together according to different types of bonds. In cereals, glucose monomers are linked via β-(1→4) and β-(1→3) bonds which make the final structure linear, while monomers in fungi and yeasts are linked via β-(1→3) and β-(1→6) bonds, resulting in a branched structure of the macromolecule. The greater complexity of the structure makes insoluble BGs more active in the organism, especially with regard to their immunostimulating and cicatrizing action, since they are able to bind better to the cellular receptors through which they exert their activities.

Preferably, according to the invention, the β-glucan used is a water-insoluble β-glucan (BG) unlike those present, for example, in barley. More preferably, the β-glucan (BG) is derived from Saccharomyces cerevisiae, which is rich in nucleotides, which are necessary for cellular development and healing.

Advantageously, β-glucan powder has properties that stimulate the immune system and increase physical resistance, but above all it develops a healing and cicatrizing action on gastric ulcers.

Advantageously, the food supplement composition according to the invention further comprises an amount of dried St. John's wort (Hypericum perforatum) flowers.

Dried St. John's wort (Hypericum perforatum) flowers have anxiolytic and musclerelaxant properties that aid digestion and relaxation.

St. John's wort (Hypericum perforatum) flowers contain various active substances, including:

- hypericin, which has antiviral and mood-enhancing properties;
- hyperforin, which has antibacterial properties and an antidepressant and anxiolytic effect;
- tannins, which have an anti-inflammatory, astringent, antidiarrheal and antiseptic action on the gastric mucosa and therefore a beneficial effect in the case of gastrointestinal disorders;
- flavonoids, particularly rutin, which have an antioxidant action and promote blood circulation;
- butyric acid, particularly gamma-amino butyric acid (GABA), which stimulates the calming effect.

The combination of rutin, hypericin and hyperforin is the basis of the anxiolytic effect of St. John's wort flowers, as it inhibits the reuptake of neurotransmitters such as serotonin and noradrenaline at the neuronal level. By doing so, a prolonged activity of these neurotransmitters is stimulated which leads to a state of inner calm and relaxation.

The food supplement composition according to the invention further comprises an amount of powder of fenugreek (Trigonella foenum-grecum) seeds.

The powder of fenugreek (Trigonella foenum-grecum) seeds has, advantageously, beneficial effects on the gastrointestinal system, facilitates digestion, causes relaxation of the muscles of the gastrointestinal tract, absorbs toxins, reduces inflammation, creates a mucilage that coats the gastric and intestinal walls, stimulates the appetite, and acts as a natural anabolic, increasing the animal's muscle development and energy levels.

The food supplement composition according to the invention further comprises an amount of powder of carob (Ceratonia siliqua) pods.

The powder of carob (Ceratonia siliqua) pods has the ability to stimulate appetite and is able to aid digestion and, advantageously, has a flavor that is highly appreciated by horses.

Optionally, the mixture may comprise an amount of flaxseed powder, which, when dissolved in water, transforms into a more or less dense mucilage that is useful for the animal's entire gastrointestinal system.

The raw materials used for the mixture according to the invention are preferably of high purity to guarantee the right content of active ingredients and organoleptic properties typical of each constituent.

For fenugreek, sweet potato, carob pod powder and St. John's wort flowers, a purity as close to 100% as possible is preferable, and even more preferably 100%.

For $\beta$-glucan (BG), a minimum purity of 75% is preferable.

Below are provided some tables containing the main properties of the ingredients constituting an embodiment example of the food supplement composition according to the invention, the results obtained from the administration of said composition to some horse specimens being provided below.

| Analytical properties of carob pod powder | |
|---|---|
| Purity | 100% |
| Particle size (micron) | $\leq$75 |
| Humidity (%) | $\leq$7% |
| Foreign matter | None |

| Analytical properties of $\beta$-glucan (BG) | |
|---|---|
| Purity | 75% |
| Particle size (micron) | $\leq$177 |
| Humidity (%) | $\leq$8% |
| Foreign matter | None |

| Analytical properties of sweet potato | |
|---|---|
| Purity | 100% |
| Particle size (micron) | $\leq$170 |

(continued)

| Analytical properties of sweet potato | |
|---|---|
| Humidity (%) | ≤12% |
| Foreign matter | None |

| Analytical properties of St. John's wort flowers | |
|---|---|
| Purity | 100% |
| Particle size (micron) | ≤170 |
| Humidity (%) | ≤ 12% |
| Foreign matter | None |

| Analytical properties of fenugreek seeds | |
|---|---|
| Purity | 100% |
| Particle size | In seeds to grind |
| Humidity (%) | ≤8% |
| Foreign matter | None |

According to a preferred embodiment of the invention, the dehydrated sweet potato powder is provided in a percentage between 25 and 50 % by weight of the mixture, the β-glucan (BG) powder is provided in a percentage between 0.5 and 3.2 % by weight of the mixture, the powder of dried St. John's wort (Hypericum perforatum) flowers is provided in a percentage between 0.5 and 3 % by weight of the mixture, the fenugreek (Trigonella foenum-grecum) powder is provided in a percentage between 20 and 70 % by weight of the mixture, and the carob (Ceratonia siliqua) pod powder is provided in a percentage between 2 and 7 % by weight of the mixture.

Optionally, the composition according to the invention may comprise a certain amount of flaxseed, in a percentage preferably not higher than 40 %.

Advantageously, the powder composition according to the invention is used as dietary supplement for horses or supplement feed. The periodic administration of this composition has both a curative effect on gastritis and inflammation of the gastrointestinal tract of horses, and a preventive effect avoiding the onset of these pathologies and also stimulating the correct functioning of the digestive system of horses.

The administration of the composition according to the invention can advantageously take place by adding the expected quantity of powder mixture of the composition according to the invention to the normal animal feed, with the addition of water, preferably at room temperature.

Furthermore, advantageously, as the composition is made up of natural ingredients, it does not present any contraindications to frequent use with unwanted side effects.

According to a first embodiment example of the invention, the powder composition will be made up as follows:

- 53.3 % of fenugreek seed powder;
- 38.5 % of sweet potato powder;
- 4.6 % of carob pod powder;
- 1.9 % of β-glucan (BG) powder;
- 1.7% of St. John's wort flowers.

In another embodiment example of the invention, the composition will be made up as follows:

- 30 % of fenugreek seed powder;
- 30 % of sweet potato powder;
- 2 % of carob pod powder;
- 0.5 % of β-glucan (BG) powder;
- 0.5 % of St. John's wort flowers;
- 37 % of flaxseed powder.

With reference to the first example given, taking into consideration a uniform sample of 3000 g of the above-mentioned powder composition, it will be composed as follows:

- 1600 g of fenugreek seed powder;
- 1154 g of sweet potato powder;
- 140 g of carob pod powder;
- 56 g of $\beta$-glucan (BG) powder;
- 50 g of St. John's wort flowers.

An exemplary dosage for the administration of the formulation according to the invention is represented by a daily dose of 150 g of the above-mentioned composition, in addition to the normal diet. This involves, in particular, the administration of 80 g/day of fenugreek seed powder, 57.7 g/day of sweet potato powder, 7 g/day of carob pod powder, 2,8 g/day of $\beta$-glucan powder and 2.5 g/day of St. John's wort flowers.

The composition thus obtained, being composed of ingredients with topical action (fenugreek seed, sweet potato, BG and carob pod powder), directly treats inflammation of the digestive system, typical of gastritis and gastric ulcers, directly alleviating the pain that afflicts animals.

Still advantageously, the composition is able to act on a psychological and emotional basis, thanks to the use of St. John's wort flowers. Animals, whose diet is naturally low in carbohydrates, are certainly not able to hydrolyze the substance $\beta$-glucan (BG), which therefore reaches both the stomach and the intestine intact. This aspect is fundamental, especially at the gastric level, since BG, not being digested, is able to exert all its activities. In doing so, in addition to stimulating the production of mucin, which is known to be present in the mucous secretions of the respiratory and gastrointestinal tracts and has the task of lubricating the tissues and protecting them from chemical and bacterial agents, BG comes into direct contact with the mucus cells and damaged tissues, promoting cicatrization thereof.

As mentioned, fenugreek seed powder and sweet potato powder stimulate the formation of a viscous film inside the stomach. Said viscous film coating the gastric walls traps the $\beta$-glucan, which is a polysaccharide, and thereby prolongs the contact time between the cells of the gastric walls and the $\beta$-glucan itself, thus allowing greater stimulation of goblet cells and faster cicatrization of damaged tissues, and therefore offering a quicker solution to the problem.

The combined use of sweet potato powder, fenugreek seed powder and $\beta$-glucan generates a synergic effect, which allows reducing the amount of $\beta$-glucan and the times of administration thereof.

In the absence of this synergistic effect, larger quantities of $\beta$-glucan (BG) and longer administration times would be necessary to obtain the same result.

Advantageously, the use of carob pod powder gives a sweet flavor, which is particularly appreciated by horses, thus encouraging the administration of the animal food composition according to the invention.

In addition, the organoleptic properties of fenugreek contribute to determining benefits at a gastrointestinal level.

Advantageously, as fenugreek seed powder is easily digestible, it determines a relaxation of the muscles of the gastrointestinal tract, and thanks to its ability to create a protective film that covers and protects the gastrointestinal walls, it reduces inflammation and its onset.

Still advantageously, fenugreek seed powder turns out to be a good natural anabolic agent, capable of increasing the animal's muscle development and its energy levels, contributing to a rapid recovery of shape of the animal that has suffered debilitation as a consequence of gastritis.

Advantageously, the powder composition according to the invention, being composed of natural elements, can be obtained at a low cost.

**In** another aspect, the present invention also relates to a method for producing the aforementioned mixture. The ingredients used for the mixture according to the invention are preferably dried at a temperature and for a time sufficient to reach the conditions that allow prolonged conservation of the product, without altering the organoleptic properties of the individual ingredients, and to avoid the denaturation of active components, such as proteins. From tests carried out, a temperature of about 63°C and a duration of about 12 hours for the drying step allows the desired objectives of prolonged conservation to be achieved, without altering the organoleptic properties of the ingredients and without the denaturation of the active components, such as the proteins contained therein.

Still according to the invention, however, it will be possible to vary these temperature and duration values according to the relative humidity RH% of the individual ingredients.

Ingredients such as $\beta$-glucan (BG), St. John's wort flowers, sweet potato and carob pod powder are generally available on the market in powder form. Fenugreek seeds, which are generally used as raw forage or for sowing, are found in granular form, whereby, in order to obtain a powder of the desired grain size, suitable for the purpose, it is necessary to grind them, for example by using a mill or a grinder, until obtaining a powder with a suitable diameter, preferably of less than 710 $\mu$m. Fenugreek seed powder, after being ground, can preferably be sieved to obtain a more uniform grain size. A 26 mesh sieve has proved to be a suitable choice.

The method for manufacturing the food supplement composition in powder form, according to the invention, comprises the steps of:

a) Weighing the individual ingredients that will make up the powder mixture for horses;
b) Adding the individual ingredients, in the amounts provided according to the invention, into a container, preferably a plastic or metal container;
c) Mixing the ingredients by means of a propeller mixer until a homogeneous powder is obtained;
d) Weighing the homogeneous powder thus obtained and filling the powder, preferably into individual packages, such as, for example, into plastic bags;
e) Adding, preferably, a graduated measuring cup inside the package;
f) Hermetically sealing the package;
g) Labelling the package.

The resulting product advantageously appears in the form of a homogeneous powder, beige/brownish in color, with a characteristic odor.

The product should preferably be kept away from light and heat to preserve its organoleptic properties and therefore the package should preferably be opaque to protect the product from light rays.

A peculiar characteristic of the mixture described above is the presence of the fenugreek seed powder with a grain size that can vary from 75 $\mu$m to 710 $\mu$m, which plays a fundamental role as an aggregating element for all the other components which have a grain size smaller than fenugreek seed powder and thanks to their own humidity bind to the larger grains of fenugreek seed powder, thereby obtaining a homogeneous powder composition.

A clinical evaluation was performed on horses subjected to a feeding cycle with the powder mixture for equines of the present invention in addition to the usual feeds administered.

The formulation of the mixture used is the mixture obtained according to the preferred embodiment, which consists of a uniform sample of 3000 g of the aforementioned powder composition for equines, composed as follows:

- 1600 g of fenugreek seed powder;
- 1154 g of sweet potato powder;
- 140 g of carob pod powder;
- 56 g of $\beta$-glucan (BG) powder;
- 50 g of St. John's wort flowers.

Seven horses, aged between 9 and 17 years, employed for competitive activities were evaluated; three female horses (mares) and four castrated male horses (geldings).

At the start of the clinical evaluation, all the evaluated subjects presented the classic symptoms of gastritis, such as: loss of coat shine, poor appetite, pain upon palpation in the gastric region, muscular tension in the trunk, refusal to work, defensiveness to the rider's requests and high nervousness during meals.

The evaluated subjects were administered a daily dose of 150 g of the above powder mixture for a total of 40 days.

| Evaluated subjects | Initial weight at day 1 (kg) | Initial weight at day 40 (kg) | Increase |
| --- | --- | --- | --- |
| Mare 1 | $\approx 500$ | 560 | +60 |
| Mare 2 | $\approx 540$ | 590 | +50 |
| Mare 3 | $\approx 500$ | 550 | +50 |
| Gelding 1 | $\approx 550$ | 620 | +70 |
| Gelding 2 | $\approx 580$ | 640 | +60 |
| Gelding 3 | $\approx 550$ | 600 | +50 |
| Gelding 4 | $\approx 540$ | 610 | +70 |

The first results were appreciated after about 15 days of administration of the mixture, while 30-40 days were necessary to achieve the maximum result. Beyond 30-40 days no significant weight variations were appreciated in the animals.

At the end of the treatment, all the evaluated horses showed a significant increase in weight, which returned to that typical of sport horses.

The horses performed consistently better after the start of clinical evaluation than in the initial situation, in which they presented acute forms of gastritis; in fact, at the end of the treatment they did not show any gastritis symptoms. In particular, it was observed that the horses' coat was shiny, the horses showed a greater propensity to work and greater energy and no symptoms of loss of appetite.

No side effects, no adverse reactions occurred, and no horse refused to eat the above powder mixture.

A further objective of the experiment was to detect the absence of forms of gastritis in subjects who fed the powder mixture every other month.

All horses tested during the experiment were fed for one year with the same amount of mixture (75g) daily and every other month.

All those horses, which in the meantime had been used for competitive activities and therefore were continuously subjected to stress, did not present any form of gastritis or ulceration during said year.

The administration of the powder mixture according to the invention has led to a very effective synergistic result in the treatment and prevention of gastritis in horses.

Based on the experimental results obtained, it was possible to define preferred effective cycles of administration of the mixture according to the invention as follows:

1) In case of a subject with acute gastritis problems:

- administering the product at full dosage for 40 days;
- administering a half dosage for 1-2 months;
- suspension of administration for one month.

2) In case of a subject with mild gastritis problems or with no gastritis:

- administering the product at half dosage every other month, with the possibility of slightly increasing the dosage in case of periods of particular stress (such as, for example, the competition season).

The food composition and method of producing the same as described and illustrated are susceptible to numerous variations and modifications falling within the same inventive principle.

## Claims

1. Food supplement composition for use in a method of preventing the onset or treating the symptoms of gastritis in equines, said method comprising a step of administering a therapeutically effective amount of the composition to an equine, wherein the composition comprises:

   - an amount of powder of dehydrated sweet potato between 25 and 50% by weight of the composition;
   - an amount of powder of $\beta$-glucan (BG) between 0.5 and 3.2 % by weight of the composition;
   - an amount of powder of dried St. John's wort (Hypericum perforatum) flowers between 0.5 and 3 % by weight of the composition;
   - an amount of powder of fenugreek (Trigonella foenum-grecum) seeds between 20 and 70 % by weight of the composition;
   - an amount of powder of carob (Ceratonia siliqua) pods between 2 and 7 % by weight of the composition.

2. Composition according to claim 1, wherein the $\beta$-glucan (BG) is a water-insoluble $\beta$-glucan (BG), more preferably a $\beta$-glucan (BG) obtained from Saccharomyces cerevisiae.

3. Composition according to claim 1 or 2, wherein said amount of powder of dehydrated sweet potato is 30 % by weight of the composition.

4. Composition according to claim 1 or 2 or 3, wherein said amount of powder of $\beta$-glucan (BG) is 0.5 % by weight of the composition.

5. Composition according to any one of the preceding claims, wherein said amount of powder of dried St. John's wort (Hypericum perforatum) flowers is 0.5 % by weight of the composition.

6. Composition according to any one of the preceding claims, wherein said amount of powder of fenugreek (Trigonella foenum-grecum) seeds is 30% by weight of the composition.

7. Composition according to any one of the preceding claims, wherein said amount of powder of carob (Ceratonia siliqua) pods is 2 % by weight of the composition.

8. Composition according to any one of the preceding claims, wherein the composition comprises an amount of flaxseed powder.

9. Supplement feed for equines, **characterized in that** it comprises a mixture of ingredients comprising a food composition according to any one of the preceding claims.

10. Feed according to claim 9, wherein 3000 g of the composition comprise:

   - 1600 g of powder of fenugreek seeds;
   - 1154 g of powder of sweet potato;
   - 140 g of powder of carob pods;
   - 56 g of powder of $\beta$-glucan (BG);
   - 50 g powder of St. John's wort flowers.

11. Method of producing the supplement feed for equines according to any one of the preceding claims, the method comprising the steps of:

   a) Weighing the individual ingredients that will make up the powder mixture for equines;
   b) Adding the individual ingredients, in the amounts provided according to the invention, into a plastic or metal container;
   c) Mixing the ingredients by means of a propeller mixer until a homogeneous powder is obtained;
   d) Weighing the homogeneous powder thus obtained and filling the powder into plastic bags;
   e) Adding a graduated measuring cup inside the plastic bag;
   f) Hermetically sealing the plastic bag;
   g) Labelling the plastic bag.

12. Method according to claim 11, wherein said ingredients are dried at a temperature and for a time sufficient to reach the conditions that allow for prolonged storage of the product, without altering the organoleptic properties of the individual components.

13. Method according to claim 12, wherein drying takes place at a temperature of about 63°C over a period of approximately 12 hours.

Europäisches
Patentamt

European
Patent Office

Office européen
des brevets

# EUROPEAN SEARCH REPORT

**Application Number**

EP 25 18 3743

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2006/008509 A1 (BEDDING PETER M J [GB] ET AL) 12 January 2006 (2006-01-12) <br> * claims 1,7 * <br> * paragraphs [0028], [0035], [0041] * <br> ----- | 1-13 | INV. <br> A23K20/163 <br> A23K50/20 <br> A23K10/30 <br> A61K31/716 |
| A | DE 20 2014 002107 U1 (ALMAPHARM GMBH & CO KG [DE]) 20 March 2014 (2014-03-20) <br> * claims 3,9 * <br> * paragraphs [0006], [0025], [0026] * <br> ----- | 1-13 | A61K36/39 <br> A61K36/48 <br> A61P1/04 |

**TECHNICAL FIELDS SEARCHED (IPC)**

A23K
A61K
A61P

The present search report has been drawn up for all claims

| Place of search | Date of completion of the search | Examiner |
|---|---|---|
| The Hague | 27 November 2025 | van Klompenburg, Wim |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

........................................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 18 3743

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2025

| Patent document cited in search report | Publication date | Patent family member(s) | | Publication date |
|---|---|---|---|---|
| US 2006008509 A1 | 12-01-2006 | AR | 044271 A1 | 07-09-2005 |
| | | AU | 2004238232 A1 | 25-11-2004 |
| | | BR | PI0410131 A | 16-05-2006 |
| | | CA | 2522765 A1 | 25-11-2004 |
| | | CN | 1794976 A | 28-06-2006 |
| | | CO | 5690535 A2 | 31-10-2006 |
| | | DK | 1626706 T3 | 20-04-2015 |
| | | EG | 25067 A | 26-07-2011 |
| | | EP | 1626706 A2 | 22-02-2006 |
| | | JO | 3001 B1 | 05-09-2016 |
| | | JP | 5085131 B2 | 28-11-2012 |
| | | JP | 5583655 B2 | 03-09-2014 |
| | | JP | 2007501632 A | 01-02-2007 |
| | | JP | 2012100672 A | 31-05-2012 |
| | | MX | PA05012050 A | 23-06-2006 |
| | | NZ | 543965 A | 26-02-2010 |
| | | PE | 20050105 A1 | 20-02-2005 |
| | | PL | 1626706 T3 | 31-08-2015 |
| | | SG | 172480 A1 | 28-07-2011 |
| | | US | 2004224036 A1 | 11-11-2004 |
| | | US | 2006008509 A1 | 12-01-2006 |
| | | US | 2011045107 A1 | 24-02-2011 |
| | | UY | 28311 A1 | 30-06-2004 |
| | | WO | 2004100826 A2 | 25-11-2004 |
| DE 202014002107 U1 | 20-03-2014 | NONE | | |

EPO FORM P0459

For more details about this annex : see Official Journal of the European Patent Office, No. 12/82

**EP 4 674 279 A1**

**Patent documents cited in the description**

- US 2006008509 A1
- DE 202014002107 U1